# EUROPEAN PATENT APPLICATION

(11) **EP 2 428 585 A1**
(43) Date of publication of application: **14.03.2012**
(21) Application number: 10771954.4
(22) Date of filing: 20.04.2010
(51) Int. Cl.: C12Q 1/68

(54) **UNIVERSAL TAGS, PROBES AND DETECTION METHODS FOR MULTIPLE TARGETS DETECTION OF BIOMOLECULE**

(30) Priority: 08.05.2009 CN 200910083561
(71) Applicant: Suzhou Institute Of Nano-Tech And Nano-Bionics Chinese Acedemy of Sciences, Suzhou, Jiangsu 215125 (CN)
(72) Inventor: LI, Jiong, Suzhou Jiangsu 215125 (CN); DUAN, Demin, Suzhou Jiangsu 215125 (CN); ZHENG, Kexiao, Suzhou Jiangsu 215125 (CN); CAO, Rong, Suzhou Jiangsu 215125 (CN); JIANG, Li, Suzhou Jiangsu 215125 (CN); LV, Zhuoxuan, Suzhou Jiangsu 215125 (CN); BAO, Fang, Suzhou Jiangsu 215125 (CN); GU, Weibing, Suzhou Jiangsu 215125 (CN)
(74) Representative: Epping - Hermann - Fischer
(86) International application number: PCT/CN2010/000541
(87) International publication number: WO 2010/127550

(57) **Abstract**

The present invention provides universal tags, probes and detection methods for multiple targets detection of biomolecules. The universal tag in the present invention is a fragment of DNA, RNA, peptide nucleic acid, or LNA, and is 3-20mer in length. The probe in the present invention contains in order from 3' terminus to 5' terminus, a nucleotide sequence which is reverse complementary to a target molecule or a portion of the target molecule, and a nucleotide sequence which is reverse complementary to the universal tag; or said probe contains in order from 3' terminus to 5' terminus, a nucleotide sequence which is reverse complementary to the universal tag, and a nucleotide sequence which is reverse complementary to a target molecule or a portion of the target molecule.

## Description

### FIELD OF THE INVENTION

The present invention relates to the field of the detection technology for biomolecules, particularly, the present invention relates to universal tags, probes and detection methods for multiple targets detection of biomolecules.

### BACKGROUND OF THE INVENTION

With the completion of human genome project (HGP), a large number of genome sequences of animals, plants and microorganisms have been determined and the gene data are increasing in an unprecedented speed. Considering that the number of genes is enormous, how to research the biology information of genes in large scale and to analyze their functions during the life process simultaneously have become a hot subject for scientists and researchers. Under the background described above, biochips based on gene chip techniques have been developed and have been deemed as one of the most significant progresses of technology since the middle of 1990s [1-4]. Gene chips, also named as DNA chips, DNA microarrays, or oligonucleotide arrays, refer to 2-dimensional DNA probe microarrays generated by using techniques of in situ synthesis or micro-spotting to fix hundreds or thousands of DNA probes on the surface of solid phase supports. Then the DNA probe microarrays are hybridized with labeled sample according to the principle of nucleic acid hybridization so that the detection and analysis of the biological specimen can be achieved quickly, in parallel, and efficiently by detecting hybridization signals. So far, gene chip techniques have been widely used in the molecular biology, the medical research and so on, and have shown a good prospect of application in the fields such as gene expression, single nucleotide polymorphism (SNP), genome research, disease diagnosis, and drug screening and so on.

Although gene chips have shown outstanding superiority in multiple, quick and parallel detection of sample molecules, there are some bottleneck factors limiting the practical application and popularization of gene chips. An important factor is that the samples to be tested need to be labeled before hybridization, and the steps of labeling samples are tedious and need to be operated by professionals. During Labeling process, reverse transcriptases, polymerases and etc. have to be used. The labeling efficiency is relatively low and the process can not be performed on the detection site. All of these factors have increased the detection cost and the operation steps and are disadvantageous to the popularization and the practical application of chip techniques. The object of the present invention is to overcome the one or more defects of current labeling methods, and to find a convenient and quick detection method of multiple targets universal tag for biomolecules.

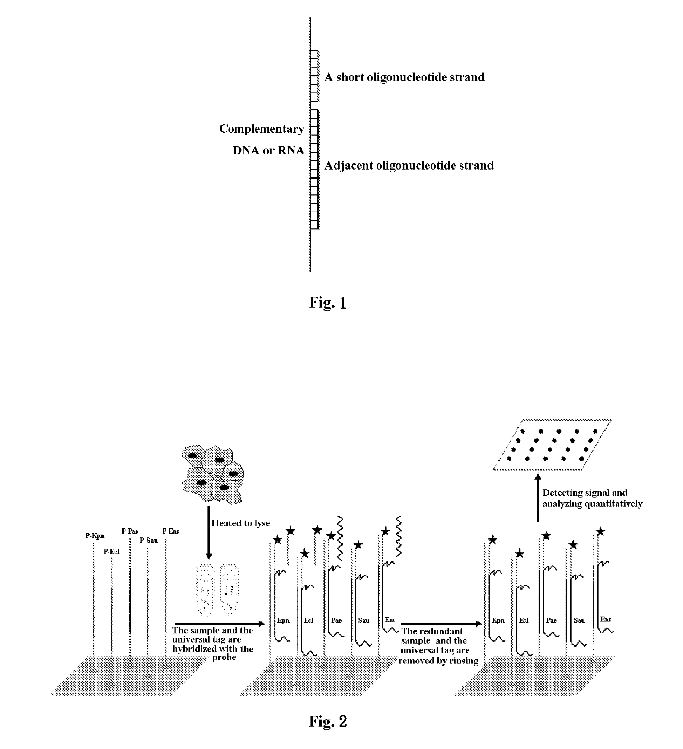

It is well known that there are two main factors for stabilizing nucleic acid double-helixes. One of the factors is the hydrogen bonds formed between the complementary base pairs, which mainly maintains the transversal stability of nucleic acid double-helixes. Another is the effect of the base stacking between the adjacent bases located on the same nucleic acid chain, which is the major factor for maintaining longitudinal stability of nucleic acid double-helixes. The two factors are functioning synergically to maintain the stability of nucleic acid double-helixes, wherein the forming of hydrogen bonds is helpful to the base stacking, while the base stacking is also helpful to the forming of hydrogen bonds. A research team led by Mirzabekov (Deceased), an academician of Russia's National Academy of Sciences, has systematically studied and explained the theory of base stacking hybridization (BSH) [5-9]. Base stacking hybridization is also named as contiguous stacking hybridization (CSH), and refers to that, when a short oligonucleotide single strand hybridizes with a complementary DNA/RNA long chain, the formed double-strand structure is usually unstable. However, if another oligonucleotide single strand adjacent to the short oligonucleotide single strand also hybridizes with the complementary DNA/RNA long chain, the stability of such double-strand structure will be greatly increased (Figure 1). Based on the research results described above, the present invention provides a universal labeling method for multiple targets detection of biomolecules.

### DESCRIPTION OF THE INVENTION

An object of the present invention is to provide a universal tag for multiple targets detection ofbiomolecules.

Another object of the present invention is to provide a probe for multiple targets detection ofbiomolecules.

Another object of the present invention is to provide a multiple targets detection method of biomolecules.

The universal tag for multiple targets detection of biomolecules according to the present invention may be a fragment of DNA, RNA, PNA (peptide nucleic acids), LNA (Locked nucleic acids) and so on. The length of the universal tag varies in the range of 3-20mer. On designing, the base sequence of the universal tag should be compared with that of the samples to be tested to avoid having the homology with the samples to be tested as far as possible.

The probe for multiple targets detection of biomolecules according to the present invention contains in order from 3' terminus to 5' terminus, a nucleotide sequence which is reverse complementary to the target molecule or a portion of the target molecule, and a nucleotide sequence which is reverse complementary to the universal tag, and a fragment of poly (T) or poly (A) optionally added on the 3' terminus to reduce the interface influence of the solid phase support. Alternately, the probe contains in order from 3' terminus to 5' terminus, a nucleotide sequence which is reverse complementary to the universal tag, a nucleotide sequence which is reverse complementary to the target molecule or a portion of the target molecule, and a fragment of poly (T) or poly (A) optionally added on the 5' terminus to reduce the interface influence of the solid phase support.

In the probe according to the present invention, the terminal group is amino, thiol, carboxyl, or biotin etc.

The multiple targets detection method ofbiomolecules according to the present invention comprises following steps:
1) preparing the universal tags, wherein the universal tags may be labeled with indicators such as fluorescent dye, quantum dot, nanogold, isotope, and biotin etc, so that they are suitable to be detected by means of fluorescence microscope, array scanner, silver staining coloration method, enzyme reaction coloration method etc;
2) preparing the probe described above, wherein, firstly the probe is designed depending on the target to be detected, and the probe contains a fragment of nucleotide sequence which is reverse complementary to the above universal tags in addition to a fragment of nucleotide sequence which is reverse complementary to the target molecule or a portion of the target molecule. The terminus of the probe is modified in order to connect with the solid phase support;
3) linking the probe to a modified solid phase support;
4) dissolving the universal tags and the sample to be tested which has been treated into a hybridization solution, hybridizing the universal tags and the sample with the probe array. Alternately, the process can be performed in two steps, i.e., hybridizing the sample to be tested with the probe, rinsing the sample, then hybridizing the universal tags with the probe array;
4) rinsing to remove the redundant sample and the redundant universal tags;
5) detecting and analyzing the hybridization signals.

In the method according to the present invention, the subject to be detected includes not only DNAs and RNAs, but also proteins, saccharide molecules, etc.

In the method according to the present invention, the solid phase support can be glass slide, plastic substrate, silicon wafer, microbeads, or polymer membrane, etc.

In the method according to the present invention, the solid phase support may be modified with poly-L-lysine, aldehyde group, carboxyl, or thiol, etc.

When detection is performed by using the universal tags and probes of the present invention, the detection can be performed directly without labeling after a sample is obtained, which greatly reduces the cost and is beneficial for the detection in situ. The experimental procedure is simplified and a nonprofessional can operate since it is easy to operate, so it is convenient for the popularization of the technology. Furthermore, multiple targets detection of biomolecules can be achieved by using the tags and the probes of the present invention.

### BRIEF DESCRIPTION OF THE DRAWINGS

Figure 1 is a schematic diagram illustrating the base stacking hybridization.
Figure 2 is a schematic diagram illustrating the universal labeling method used in the detection of various clinical pathogens.
Figure 3 is a schematic diagram illustrating the universal labeling method used in the analysis of miRNA profile.
Figure 4 is a schematic diagram illustrating the universal labeling method used in the multiple detection of protein targets.
Figure 5 is a schematic diagram illustrating the universal labeling method used in the analysis of miRNA profile.

### DETAILED DESCRIPTION OF THE EMBODIMENTS

### Example 1. Application of the method according to the present invention in the detection of various clinical pathogens.

Five pathogens obtained from respiratory passage of pneumonia are taken as examples(shown in figure 2) to describe the Example 1: *K.pneumoniae, E.cloacae, P.aeruginosa, S.aureus,* and *Enterococcus.* The probes and universal tags to be used are shown in Table 1.

**Table 1. Names and sequences of the probes and the universal tag used in the detection of the five pathogen samples obtained from respiratory passage of pneumonia**

| Probes | targets | Sequences (5'-3') |
|---|---|---|
| P-Kpn | *K.*pneumoniae 16SRNA | NH2-T12-AACCGCTGGCAACAAAG-TACGACACT |
| P-Ecl | *E.cloacae* 16SRNA | NH2-T12-GTAGGTAAGGTTCTTCG-TACGACACT |
| P-Pae | *P.aeruginosa* 16SRNA | NH2-T12-GCGCCCGTTTCCGGAC-TACGACACT |
| P-Sau | *S.aureus* 16SRNA | NH2-T12-AGCAAGCTTCTCGTCCG-TACGACACT |
| P-Enc | *Enterococcus* 16SRNA | NH2-T12-GTTTCCAAGTGTTATCCC-TACGACACT |
| universal tag | | |
| UT-16S | | FAM-AGTGTCGTA |

| | | |
|---|---|---|
| 1. 16SRNAs of the five pathogens are chosen as the targets of detection, and five probes are synthesized, respectively, wherein the 5' terminus of the probe is poly (T) 12, a fragment of sequence in middle of the probe is complementary to a portion of the target molecule, a fragment of sequence on the 3' terminus is complementary to the universal tag, and the 5' terminus of the probe is modified with amino group; 2. The universal tag is synthesized and is modified with fluorescein on its 5' terminus; 3. A glass slide is treated by using conventional chemical modification method to prepare an aldehyde substrate; 4. The probe is dissolved in a spotting buffer solution, and then the oligonucleotide arrays are prepared by spotting; 5. The secretion substance from respiratory passage of a patient is heated to lyse , or the bacterial culture suspension is heated to lyse after the secretion substance is bacterial-cultured. Then the lysed substance is dissolved in hybridization solution together with the universal tag and hybridized with the probe arrays; 6. The redundant samples and the redundant universal tag are removed by rinsing; 7. The detection is performed by using fluorescence microscope or array scanner and analysis is performed. | | |

Because of the effect of base stacking hybridization, the universal tag can be linked to the probes steadily only when the completely complementary target molecules are linked to the probes. When the mismatched target molecules are linked to the probes, the linkage between the universal tag and the probes can not be stabilized. The five probes are hybridized with the 16SRNAs of the five pathogens, respectively. Thus the types and contents of the infected pathogens can be determined to guide clinical medication.

### Example 2. Application of the method according to the present invention in the analysis of miRNA profile.

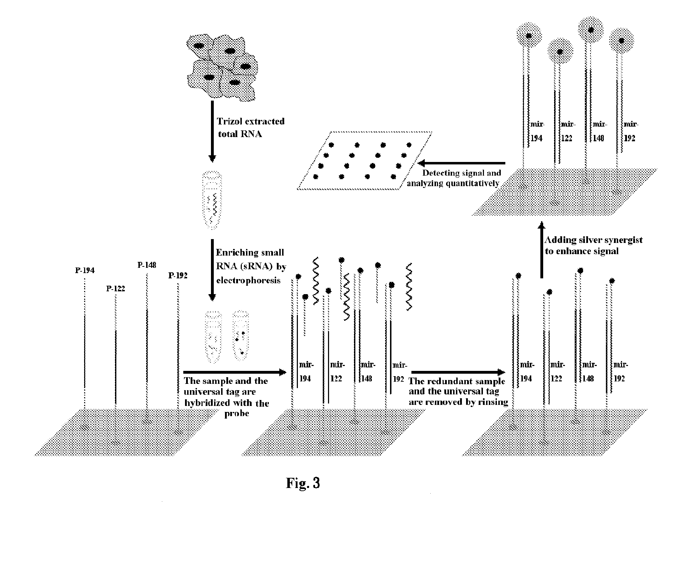

Four miRNAs obtained from tissue of liver are taken as examples (shown in figure 3) to describe the Example 2: hsa-mir-194, hsa-mir-122, hsa-mir-148, and hsa-mir-192. The probes and universal tags to be used are shown in Table 2.

**Table 2. Names and sequences of the probes and the universal tag to be used in the detection of the four miRNAs obtained from the tissue of liver**

| Probes | Targets | sequences (5'-3') |
|---|---|---|
| P-194 | hsa-mir-194 | NH2-A10-TCCACATGGAGTTGCTGTTACA-TGCGACCTG |
| P-122 | hsa-mir-122 | NH2-A10-CAAACACCATTGTCACACTCCA-TGCGACCTG |
| P-148 | hsa-mir-148 | NH2-A10-ACAAAGTTCTGTAGTGCACTGA-TGCGACCTG |
| P-192 | hsa-mir-192 | NH2-A10-GGCTGTCAATTCATAGGTCAG-TGCGACCTG |
| universal tag | | |
| UT-miRNA | | nanogold-CAGGTCGCA |

| | | |
|---|---|---|
| 1. The probes corresponding to above four miRNAs are prepared according to miRNA library, wherein the 5' terminus of the probe is poly (A) 10, a fragment of sequence in middle of the probe is complementary to the miRNA, a fragment of sequence on the 3' terminus is complementary to the universal tag, and the 5' terminus of the probe is modified with amino group; 2. The universal tag is synthesized and is modified with nanogold on its 5' terminus; 3. A glass slide is treated by using conventional chemical modification method to prepare an aldehyde substrate; 4. The probes are dissolved in a spotting buffer solution, and then the oligonucleotide arrays are prepared by spotting; 5. After the samples are lysed or total RNAs are extracted and small RNAs (sRNAs) are separated and enriched, the samples, together with the universal tag, are dissolved in a hybridization solution and hybridized with the probe; 6. The redundant samples and the redundant universal tag are removed by rinsing; 7. A silver synergist is added to enhance the signal; 8. The signals are detected and analyzed by using a flat plate scanner to determine the expression profile of the miRNA. | | |

### Example 3. Application of the method according to the present invention in multiple detection for protein targets

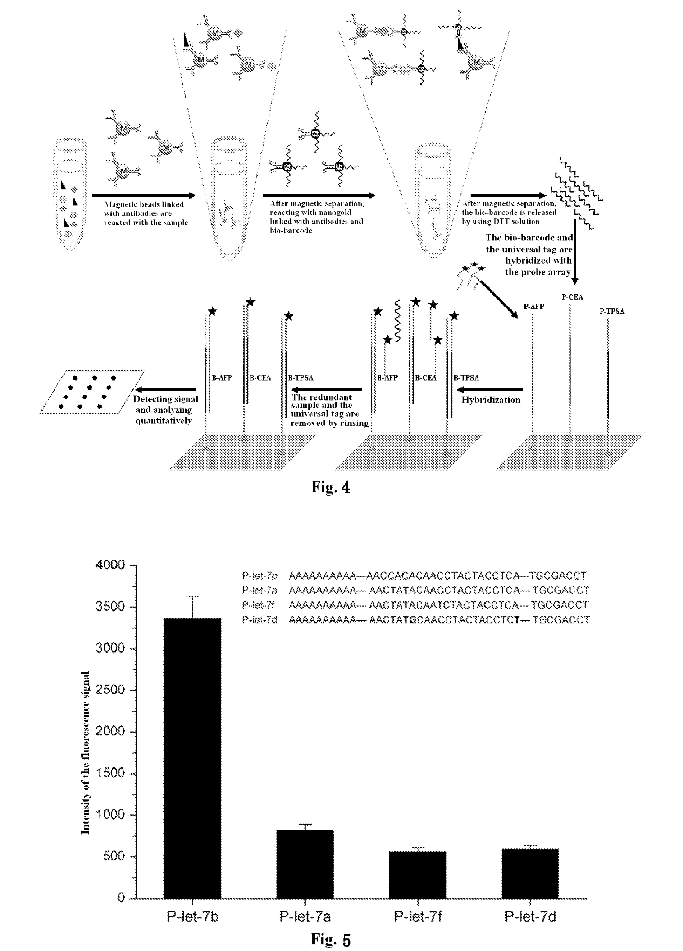

Alpha fetoprotein (AFP), carcino-embryonic antigen (CEA), and total prostate specific antigen (TPSA) which are obtained from human serum are taken as examples (shown in figure 4) to describe the Example 3. The probes, bio-barcodes, and universal tags to be used are shown in Table 3.

**Table 3. Names and sequences of the probes, bio-barcodes, and universal tag to be used in the detection of the three antigens from human serum**

| Probes | targets | sequences (5'-3') |
|---|---|---|
| P-AFP | alpha fetoprotein | NH2-T10-CAGCATCGGACCGGTAATCG- TACGACACT |
| P-CEA | carcino-embryonic antigen | NH2-T10-TGCGATCGCAGCGGTAACCT- TACGACACT |
| P-TPSA | total prostate specific antigen | NH2-T10-GACCATAGTGCGGGTAGGTA- TACGACACT |
| bio-bar code | | |
| B-AFP | alpha fetoprotein | CGATTACCGGTCCGATGCTG |
| B-CEA | carcino-embryonic antigen | AGGTTACCGCTGCGATCGCA |
| B-TPSA | total prostate specific antigen | TACCTACCCGCACTATGGTC |
| universal tag | | |
| UT-pro | | FAM-AGTGTCGTA |

| | | |
|---|---|---|
| 1. The antibodies corresponding to the three antigens to be detected are linked to magnetic beads and the magnetic beads linked with antibodies are then reacted with sample solutions, so as to form the antigen-antibody complexes; 2. The redundant samples are removed by magnetic separation, and then the nanogolds modified with the antibody and bio-barcode (the three antigens to be detected are corresponded to three different barcode nucleotide sequences), are reacted with the antigen-antibody complexes, to form the complexes of magnetic bead-antigen-nanogold; 3. The redundant nanogold is removed by magnetic separation, and then the bio-barcodes are released from nanogold by using DTT solution; 4. The released bio-barcodes and the universal tags labeled with FAM are dissolved in hybridization solution and hybridized with the probe array (the 3' terminus of the probe is complementary to the universal tag, the portion in middle of the probe is complementary to corresponding bio-barcode, the 5' terminus is poly (T) 10, and the 5' terminus is modified with amino group so as to be fixed on the aldehyde glass slide); 5. The redundant universal tag is removed by rinsing; 6. The detection and analysis are performed by using a fluorescence microscope or an array scanner to determine the types and contents of the three antigens in the serum sample. | | |

### Example 4. Application of the method according to the present invention in the analysis of miRNA. which has high specificity

Four members hsa-let-7b, hsa-let-7a, hsa-let-7f, and hsa-let-7d of hsa-let-7 family of miRNA (shown in figure 5) are taken as examples to descibe the Example 4, wherein the probes, universal tags, and targets to be used are shown in Table 4.

**Table 4. Names and sequences of the probes, universal tags, and targets used in the detection of the hsa-let-7 family**

| Names | sequences (5'-3') |
|---|---|
| probeP-let-7b | AAAAAAAAAA-AACCACACAACCTACTACCTCA-TGCGACCT |
| probeP-let-7a | AAAAAAAAAA-AACTATACAACCTACTACCTCA-TGCGACCT |
| probeP-let-7f | AAAAAAAAAA-AACTATACAATCTACTACCTCA-TGCGACCT |
| probeP-let-7d | AAAAAAAAAA-AACTATGCAACCTACTACCTCT-TGCGACCT |
| universal tag | AGGTCGCA |
| target T-let-7b | ugagguaguagguugugugguu |

| | |
|---|---|
| Note: The bases represented with black body in the table are bases mismatched with the target T-let-7b. 1. Four probes corresponding to hsa-let-7b, hsa-let-7a, hsa-let-7f, and hsa-let-7d, respectively, are synthesized according to miRNA library, wherein the 5' terminus of the probe is poly (A) 10, a fragment of sequence in middle of the probe is complementary to the relavent miRNA, a fragment of sequence on the 3' terminus is complementary to the universal tag, and the 5' terminus of the probe is modified with amino groups; 2. The universal tag is synthesized, and the 5' terminus is modified with luciferin Cy3; 3. The target T-let-7b is synthesized; 4. An aldehyde glass slide is prepared by using chemical modification method; 5. The probes are dissolved in a spotting buffer solution, and then, oligonucleotide arrays of four probes are prepared by spotting process; 6. Target T-let-7b and universal tag are dissolved in a hybridization solution and hybridized with the arrays; 7. The glass slide of arrays is rinsed; 8. The glass slide is scanned with scanner; 9. The result shows that the method of the invention has high specificity, and is able to identify targets which have only 2, 3, or 4 mismatched bases. | |

### References

[1] Fodor SP, Read JL, Pirrung MC, Stryer L, Lu AT, Solas D. Light-directed, spatially addressable parallel chemical synthesis. Science 1991 Feb 15; 251(4995): 767-773.
[2] Breakthrough of the year. The runners-up. Science.1998 Dec 18; 282(5397): 2157-2161.
[3] Marshall A, Hodgson J. DNA chips: an array of possibilities. Nat Biotechnol.1998 Jan; 16(1): 27-31.
[4] Service RF. Microchip arrays put DNA on the spot. Science. 1998 Oct 16; 282(5388): 396-399.
[5] Yershov G, Barsky V, Belgovskiy A, Kirillov E, Kreindlin E, Ivanov I, Parinov S, Guschin D, Drobishev A, Dubiley S, Mirzabekov A. DNA analysis and diagnostics on oligonucleotide microchips. Proc Natl Acad Sci U S A. 1996 May 14;93(10):4913-4918.
[6] Parinov S, Barsky V, Yershov G, Kirillov E, Timofeev E, Belgovskiy A, Mirzabekov A. DNA sequencing by hybridization to microchip octa-and decanucleotides extended by stacked pentanucleotides. Nucleic Acids Res. 1996 Aug 1;24(15):2998-3004.
[7] Dubiley S, Kirillov E, Lysov Y, Mirzabekov A. Fractionation, phosphorylation and ligation on oligonucleotide microchips to enhance sequencing by hybridization. Nucleic Acids Res. 1997 Jun 15; 25(12):2259-2265.
[8] Parallel thermodynamic analysis of duplexes on oligodeoxyribonucleotide microchips. Fotin AV, Drobyshev AL, Proudnikov DY, Perov AN, Mirzabekov AD. Nucleic Acids Res. 1998 Mar 15; 26(6):1515-1521.
[9] Vasiliskov VA, Prokopenko DV, Mirzabekov AD. Parallel multiplex thermodynamic analysis of coaxial base stacking in DNA duplexes by oligodeoxyribonucleotide microchips. Nucleic Acids Res. 2001 Jun 1; 29(11): 2303-2313.

## Claims

1. A universal tag for multiple targets detection of biomolecules, **characterized in that**,
the universal tag is a fragment of DNA, RNA, peptide nucleic acid, or LNA;
the universal tag is 3-20mer in length.

2. The universal tag according to claim 1, **characterized in that**, the universal tag is labeled with indicators including fluorescent dye, quantum dot, nanogold, isotope, and/or biotin.

3. A probe for multiple targets detection of biomolecules, **characterized in that**, the probe contains in order from 3' terminus to 5' terminus, a nucleotide sequence which is reverse complementary to a target molecule or a portion of a target molecule, and a nucleotide sequence which is reverse complementary to the universal tag ;
or, the probe contains in order from 3' terminus to 5' terminus, a nucleotide sequence which is reverse complementary to the universal tag, a nucleotide sequence which is reverse complementary to a target molecule or a portion of a target molecule,
wherein the universal tag is a fragment of DNA, RNA, peptide nucleic acid, or LNA, which is 3-20mer in length.

4. The probe according to claim 3, **characterized in that** the terminal group is amino, thiol, carboxyl, or biotin.

5. A multiple targets detection method of biomolecules, **characterized in that** the method comprises steps of:
1) preparing the universal tag according to claim 1;
2) preparing the probe according to claim 2;
3) linking the probe to a modified solid phase support;
4) dissolving the universal tag and samples to be tested into a hybridization solution to hybridize with the probe array; or, hybridizing the sample to be tested with the probe first, then hybridizing the universal tag with the probe array after rinsing;
5) rinsing to remove the redundant samples and the redundant universal tag;
6) detecting and analyzing hybridization signal.

6. The method according to claim 5, **characterized in that** the solid phase support is glass slide, plastic substrate, slice of silicon, microbead or polymer membrane.

7. The method according to claim 5, **characterized in that** the solid phase support is modified with epoxy group, amino, poly-L-lysine, aldehyde group, carboxyl, or thiol.

8. The method according to claim 5, **characterized in that** the subject to be detected in the detection method is DNA, RNA, protein and/or molecule of saccharide.
